# EUROPEAN PATENT APPLICATION

(11) **EP 1 479 409 A1**
(43) Date of publication of application: **24.11.2004**
(21) Application number: 03743982.5
(22) Date of filing: 20.02.2003
(51) Int. Cl.: A61M 25/10

(54) **BALLOON CATHETER FOR TENTATIVE VASO-OCCLUSION**

(30) Priority: 27.02.2002 JP 2002052475
(71) Applicant: Kaneka Corporation, Osaka-shi, Osaka 530-0005 (JP)
(72) Inventor: MIKI, Shogo, Suita-shi, Osaka 565-0824 (JP); NAKANO, Ryoji, Settsu-shi, Osaka 566-0074 (JP)
(74) Representative: Gille Hrabal Struck Neidlein Prop Roos
(86) International application number: PCT/JP2003/001840
(87) International publication number: WO 2003/075996

(57) **Abstract**

The present invention aims to provide an intravascular temporary occlusion balloon catheter that can itself function as a guidewire, has superior maneuverability suitable for highly tortuous or branched blood vessels such as coronary or cerebral arteries, can be inserted into the blood vessel over the guidewire, and can be sufficiently inserted into the peripheral region of the blood vessel. The present invention includes a balloon composed of a highly tensile material having an elongation at break of 300% to 1,100% and a shaft composed of a highly elastic material and having an outer diameter in the range of 0.014 in. to 0.018 in. and a bending modulus of at least 1 GPa, wherein a lumen for tracking the guidewire is provided at a catheter distal-end portion only.

## Description

### Technical Field

The present invention relates to balloon catheters that can be introduced into the body through percutaneous transluminal procedures to cause local occlusion of blood vessels. In particular, it relates to a balloon catheter that can cause temporary occlusion of blood vessels in the periphery of target disease areas so that atheromas resulting from angioplasty and thrombi in blood vessels such as the cerebral artery, carotid artery, coronary artery, coronary artery bypass grafts, renal artery, pulmonary artery, and the like do not spread to peripheral vessels.

### Background Art

When stenosis or occlusion occurs in a vessel such as a blood vessel, angioplasty (e.g., percutaneous transluminal angioplasty (PTA) or percutaneous transluminal coronary angioplasty (PTCA)) is frequently performed in many medical institutions to increase the blood flow in peripheral blood vessels by expanding the narrowed or occluded area in the blood vessel. Angioplasty has become a common procedure for treating such cases. The use of stents to maintain the expanded area has also increased in recent years.

A balloon catheter for PTA and PTCA is used with a guide catheter and a guidewire to expand the narrowed or occluded area in the blood vessel. The angioplasty using the balloon catheter is conducted as follows: The guide catheter is first inserted from the femoral artery into the aorta until the distal end of the guide catheter reaches the entrance of the coronary artery. The guidewire penetrating the balloon catheter is advanced past the narrowed or occluded area of the blood vessel, and the balloon catheter is then advanced over the guidewire until the balloon reaches the narrowed or occluded area. The balloon is inflated to expand the narrowed or occluded area, deflated, and withdrawn. The balloon catheter is effective not only for treatments of narrowed or occluded sites of blood vessels but also in other medical applications that involve insertion into blood vessels, various coeloms, and tubular tissues.

When the blood vessel occlusion is caused by thrombi, expansion of the occluded area with a balloon catheter may cause the thrombi to detach from the inner wall of the blood vessels, thereby causing occlusion of downstream peripheral blood vessels. In expanding the narrowed area of the blood vessel having a diseased area containing large numbers of atheroplaques, the atheroplaques (atheromas) may spread as a result of the balloon expansion, thereby causing occlusion of peripheral blood vessels. When occlusion of peripheral vessels occurs, blood does not flow into the peripheral vessels despite the expansion of the occluded or narrowed area. This results in a slow-flow or no-reflow phenomenon.

In the event of such a phenomenon, it is a generally accepted practice to wait for the recovery of the blood flow in, e.g., the coronary artery. However, the recovery requires time. If feasible, a vasodilator may be administered to recover the blood flow, or a drug such as a thrombolytic agent may be locally administered to dissolve the obstruction. However, this also requires time before the recovery of the blood flow can be achieved. An auxiliary technique such as intra-aortic balloon pumping (IABP) may also be employed for cases of severe peripheral occlusion and hemodynamic deterioration.

In particular, when blood vessel occlusion or stenosis occurs in the carotid artery or the cerebral artery, the peripheral occlusion resulting from the stent/balloon catheter angioplasty stops the blood flow to the brain and causes ischemia in the brain cells downstream of the occluded area. Ischemia can kill brain cells if it continues for a long period of time, and severe and permanent damage may result. Adequate care must be exercised to prevent occlusion of peripheral vessels, especially when performing angioplasty in the cerebral or the carotid arteries.

In order to prevent accidental occlusion of the peripheral blood vessels, there has been an attempt to temporarily occlude the peripheral vessels of the affected blood vessel during angioplasty.

A conventional intravascular temporary occlusion balloon catheter of a type having a guidewire function for delivering a device such as a stent, just like the present invention, includes a guidewire with a hollow shaft and a balloon attached to the distal-end portion of the guidewire. However, the balloon of a catheter of this type is located approximately 30 mm from the distal end of the guidewire, and the stiffness of the guidewire is insufficient due to the hollow catheter shaft. Accordingly, the guidewire has low maneuverability and cannot be used in highly tortuous and branched blood vessels such as coronary and cerebral arteries.

Moreover, conventional intravascular temporary occlusion balloon catheters do not have guidewire lumens. Although intravascular temporary occlusion balloon catheters can function as guidewires, it is not possible to insert such a temporary occlusion balloon catheter into the blood vessel over a conventional guidewire. The guidewire must have superior flexibility, blood vessel trackability, and stiffness in order for it to be inserted into a highly tortuous target disease area. However, insertion has been difficult even when the intravascular temporary occlusion balloon catheter is used as a guidewire.

In coronary artery angioplasty procedures, a PTCA balloon catheter or a stent is usually delivered after a guidewire has been advanced past the region of the blood vessel in which the diseased area is located. The guidewire is advanced to sufficiently reach this periphery to prevent the guidewire from losing its position in the periphery during the course of advancing the other device, such as a PTCA balloon catheter. Otherwise, it is not possible to readily deal with an occlusion of the peripheral area that may occur after the treatment of an upstream blood vessel. Because of these reasons, the guidewire is usually advanced past the diseased area of the target blood vessel into the peripheral area. However, since the conventional temporary occlusion balloon is fixed at a position approximately 30 mm from the distal end of the guidewire, the guidewire cannot be sufficiently inserted to the peripheral area while delivering the occlusion balloon near the diseased area. Moreover, it has rarely been possible to deal with an accidental occlusion of the peripheral blood vessels that might occur after deflation of the intravascular temporary occlusion balloon.

### Disclosure of Invention

The present invention aims to provide a temporary occlusion balloon catheter that can itself function as a guidewire, that has superior maneuverability suitable for highly tortuous or branched blood vessels such as coronary or cerebral arteries, that can be inserted into the blood vessel over the guidewire, and that can be sufficiently inserted into the peripheral region of the blood vessel.
[1] The present invention provides an intravascular temporary occlusion balloon catheter including a balloon composed of a highly tensile material having an elongation at break of 300% to 1,100%, and a shaft composed of a highly elastic material and having an outer diameter in the range of 0.014 in. (0.3556 mm) to 0.018 in. (0.4572 mm) and a bending modulus of at least 1 GPa, wherein a lumen for tracking the guidewire is provided at a catheter distal-end portion only.
[2] The present invention provides the intravascular temporary occlusion balloon catheter of [1], wherein the lumen for tracking the guidewire penetrates the interior of the balloon.
[3] The present invention provides the intravascular temporary occlusion balloon catheter of [2], wherein the lumen for tracking the guidewire has a proximal-side guidewire port located at a position within 10 mm to 2 mm from the proximal end of the inflated balloon.
[4] The present invention provides the intravascular temporary occlusion balloon catheter according [2] or [3], wherein the guidewire port is closed when no guidewire is inserted.
[5] The present invention provides the intravascular temporary occlusion balloon catheter according [1], wherein the lumen for tracking the guidewire is located at the distal side of the balloon.
[6] The present invention provides the intravascular temporary occlusion balloon catheter according to any one of [1] to [5], wherein the shaft of the catheter is composed of SUS 304, SUS 316, or SUS 316L stainless steel.
[7] The present invention provides the intravascular temporary occlusion balloon catheter according to any one of [1] to [6], wherein the shaft is composed of a superelastic metal at least in the distal side.
[8] The present invention provides the intravascular temporary occlusion balloon catheter according to any one of [1] to [7], wherein the outer surface of the shaft is covered with a thin resin layer composed of tetrafluoroethylene or polyethylene or a hydrophilic coating layer.
[9] The present invention provides the intravascular temporary occlusion balloon catheter according to any one of [1] to [8], further including a radiopaque marker for identifying the position of the catheter by radioscopy, the radiopaque marker being disposed at least in the interior of the balloon.
[10] The present invention provides the intravascular temporary occlusion balloon catheter according to any one of [1] to [9], wherein the balloon catheter is composed of thermoplastic polyurethane, silicone, or natural rubber.

### Brief Description of the Drawings

Fig. 1 is a cross-sectional view of the relevant part of a catheter 101 of the present invention taken in the longitudinal direction.
Fig. 2 is a cross-sectional view of a dual lumen tube 201.
Fig. 3 is a cross-sectional view of the relevant part of a catheter 301 of the present invention taken in the longitudinal direction.
Fig. 4 is a diagram showing the catheter 301 of the present invention viewed from the A-A cross-section toward the catheter distal end.
Fig. 5 is a cross-sectional view of the relevant part of a catheter 501 of the present invention taken in the longitudinal direction.
Fig. 6 is a cross-sectional view of the relevant part of a catheter 601 of the present invention taken in the longitudinal direction.
Fig. 7 is a cross-sectional view of the relevant part of a catheter 701 of the present invention taken in the longitudinal direction.
Fig. 8 is a cross-sectional view of the relevant part of a catheter 801 of the present invention taken in the longitudinal direction.

### Best Mode for Carrying Out the Invention

Embodiments of the catheter of the present invention will now be described. Note that the present invention is not limited to these embodiments.

The present invention provides an intravascular temporary occlusion balloon catheter including a balloon composed of a highly tensile material having an elongation at break of 300% to 1,100%, and a shaft composed of a highly elastic material and having an outer diameter in the range of 0.014 in. (0.3556 mm) to 0.018 in. (0.4572 mm) and a bending modulus of at least 1 GPa, in which a lumen for tracking the guidewire is provided at a catheter distal-end portion only. Here, the term "catheter distal-end portion" refers to the region extending from a position 30 mm from the balloon toward the proximal side up to a position 30 mm from the balloon toward'the distal side.

The guidewire lumen at the distal-end portion allows an operator familiar with operating the guidewire to insert the guidewire to the periphery of the diseased area and to deliver the intravascular temporary occlusion balloon catheter of the present invention over the guidewire. Since the catheter can be delivered over the guidewire, the catheter can be delivered to highly tortuous or branched areas of the coronary or cerebral arteries.

Since the guidewire is in the periphery, it is possible to readily deal with a peripheral occlusion or the like that may occur upon deflation of the balloon of the inventive intravascular temporary occlusion balloon catheter. A stent or other device is typically compatible with wires having an outer diameter of 0.014 in. (0.3556 mm) to 0.018 in. (0.4572 mm). Thus, the outer diameter of the shaft portion of the inventive catheter is adjusted in the range of 0.014 in. (0.3556 mm) to 0.018 in. (0.4572 mm) so that the shaft of the balloon catheter can function as the guidewire that guides the stent or other device along the shaft.

In order to leave the stent in place after delivery over the inventive balloon catheter, the guidewire must be pulled back to the proximal side of the stent. However, a peripheral occlusion that may occur at this time can be readily dealt with since the guidewire can be advanced again to the periphery at the same time as the deflation of the inventive balloon.

The balloon is used to produce a temporal vessel occlusion and must not damage the blood vessel during the occlusion. A balloon composed of a highly elastic material having an elongation at break of 300% to 1,100% is suitable for avoiding blood vessel damage. A material having an elongation at break of 400% to 900% is more preferred, and a material having an elongation at break of 500% to 700% is most preferred.

The shaft composed of a highly elastic material and having a bending modulus of at least 1 GPa can efficiently transmit the force of the operator to the catheter distal end. In order to transfer the force generated by pushing, pulling, and turning operations to the distal end, a highly elastic material having a bending modulus of at least 1 GPa is suitable.

The lumen for tracking the guidewire penetrates the interior of the balloon so as to decrease the resistance when the lumen tracks the guidewire. The guidewire passes through the center of the inventive balloon so that the inventive balloon and the balloon of the PTCA or PTA are concentric during insertion of the PTCA or PTA balloon catheter over the guidewire. The advantage of such an arrangement lies in the fact that the inventive balloon, which is generally inflated at a low pressure, is rarely affected by a PTCA or PTA balloon, which is generally inflated at a high pressure, even if they are inflated simultaneously.

A guidewire port, i.e., a proximal-side opening of the lumen for tracking the guidewire, is preferably located 10 mm to 2 mm away from the proximal end of the inflated balloon. In some cases, a procedure using a suction catheter or the like is performed to remove thrombi and atheromas present at the proximal side of the inventive balloon. In such an event, the suction catheter is typically delivered over the guidewire. Accordingly, if the distance between the guidewire port and the inventive balloon is excessively large, the suction catheter cannot be advanced beyond the guidewire port, thereby creating a dead zone between the balloon and the suction catheter. The suction catheter cannot remove thrombi and atheromas present in the dead zone. Thus, the guidewire port must be located within 10 mm from the proximal end of the balloon to decrease the risk of suction failure with the suction catheter. On the other hand, it is difficult to form the guidewire port in the region within 2 mm from the balloon since a particular gap must be left to bond the balloon. Thus, the guidewire port is preferably at a position in the region from 10 mm to 2 mm from the proximal end of the balloon. The more preferable region is from 5 mm to 2 mm from the proximal end to achieve better thrombus/atheroma suction performance.

The guidewire port is arranged so as to close when no guidewire is inserted therethrough in order to prevent blood from flowing to the peripheral vessels via the guidewire lumen during the stent placement. In particular, a proximal-end portion of the tube that defines the guidewire lumen is pulled to protrude toward the proximal side, and the protruded portion is squashed from the side and heated to memorize the shape. The squashed portion will function as a valve for efficiently preventing blood from flowing to the periphery when the guidewire is pulled in the proximal direction.

Since the lumen for tracking the guidewire is provided at the distal side of the balloon, blood is completely prevented from flowing to the peripheral vessels during the stent placement. Alternatively, the profile of the balloon can be reduced by not allowing the guidewire lumen to cross the interior of the balloon; however, the trackability of the guidewire is low compared to the guidewire that crosses the interior of the balloon. An appropriate selection must be made as to the type of guidewire used.

The shaft may be made of SUS 304, SUS 316, or SUS 316L stainless steel to reduce the thickness and the diameter and to improve the workability.

At least the distal-end shaft of the shaft is composed of a superelastic metal to reduce the risk of kinking of the shaft portion protruding from the distal end of the guiding catheter. Moreover, the shaft does not have a tendency to kink, resulting in an improved catheter maneuverability.

The outer surface of the shaft is coated with a thin-film resin layer composed of tetrafluoroethylene or polyethylene or with a hydrophilic coating layer to decrease the sliding friction of PTCA or PTA balloon catheters or other devices such as stent delivery catheters or suction catheters. When the shaft has a thin-film resin layer composed of tetrafluoroethylene or polyethylene, thrombi can be reduced.

A radiopaque marker for identifying the location of the catheter by radioscopy may be placed inside the balloon. In this manner, the operator can identify the precise location of the catheter.

The balloon catheter may be made of thermoplastic polyurethane, silicone, or natural rubber.

### (EXAMPLE 1)

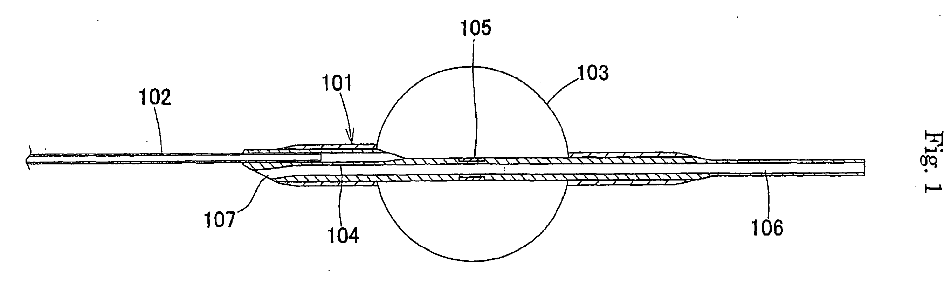

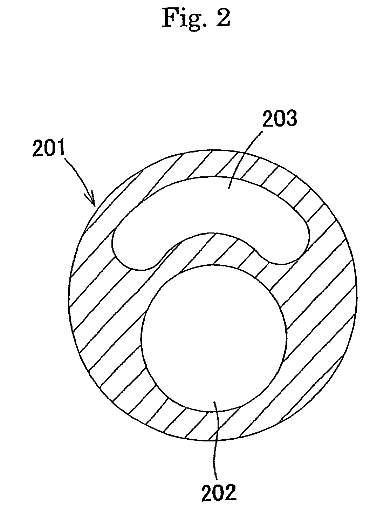

Fig. 1 shows an example of the present invention. A metal tube having a small wall thickness and a small diameter and composed of SUS 316L stainless steel was used as a shaft 102 of a catheter 101. The metal tube had an outer diameter of 0.35 mm, an inner diameter of 0.28 mm, and a length of 1,800 mm. A balloon 103 was made by a dip-forming process using a solution containing 5 percent by weight of E660 manufactured by Nippon Miractran Co., Ltd., in a tetrahydrofuran (THF) solvent. The dip-forming process was conducted by dipping a tetrafluoroethylene-coated mandrel, i.e., a core, having an outer diameter of 1.2 mm, into the prepared solution and withdrawing the mandrel so as to form a balloon tube on the mandrel. The dipping and withdrawing of the mandrel were repeated until the thickness of the balloon tube became 80 µm, which was sufficient for use as the balloon 103. A base shaft 104 for fixing the shaft 102 and the balloon 103 was prepared by processing the dual lumen tube 201 shown in Fig. 2. The dual lumen tube 201 had an outer diameter of 0.95 mm, was composed of a polyamide elastomer Pebax 7233 manufactured by Atofina Chemicals, Inc., and included a circular lumen 202 having an inner diameter of 0.50 mm and a crescent-shaped lumen 203 having a maximum width of 0.25 mm. The base shaft 104 was processed as follows: The dual lumen tube 201 was cut to a length of 20 mm. A rectangular mandrel having a thickness of 0.20 mm and a width of 0.30 mm was inserted into the crescent-shaped lumen 203, and the outer wall portion (the arc portion) of the crescent-shaped lumen 203 was cut away with a razor blade while leaving a 5-mm uncut portion at one end. This process was done to allow insertion of the dual lumen tube 201 into the crescent-shaped lumen 203 to communicate the lumen inside the dual lumen tube 201 with the interior of the balloon. Next, a tetrafluoroethylene-coated mandrel having an outer diameter of 0.40 mm and carrying a platinum radiopaque marker 105 having an outer diameter of 0.48 mm, an inner diameter of 0.42 mm, and a length of 1 mm was inserted together with the radiopaque marker 105 into the circular lumen 202 of the dual lumen tube 201. At this stage, the radiopaque marker 105 was adjusted so that the radiopaque marker 105 was located at the center of the portion where the balloon 103 was to be mounted. Subsequently, a two-part urethane adhesive was thinly applied on the part of the shaft 102 within 5 mm from the tip, and the shaft 102 was inserted into the 5-mm uncut portion of the crescent-shaped lumen 203 of the dual lumen tube 201. At this stage, a tapered mandrel was inserted to the end of the shaft 102 in advance so that the adhesive would not flow into the interior of the shaft 102. For example, the tapered mandrel may have a length of 50 mm and an outer diameter gradually changing from 0.20 mm to 0.35 mm. The two-part urethane adhesive was prepared by mixing Nippolan 4235 and Coronate 4403 manufactured by Nippon Polyurethane Industry Co., Ltd., at a 2:1 ratio. When the shaft 102 with the applied adhesive was inserted into the crescent-shaped lumen 203 of the dual lumen tube 201, the outer wall of the crescent-shaped lumen 203 was stretched, and the cross-section of the dual lumen tube 201 became elliptic in this region. While maintaining this state, a heat-shrinkable tube was placed so as to wrap the portion of the dual lumen tube 201 that would form the base shaft 104 and heated to form the base shaft 104 integrated with the shaft 102. The heat-shrinkable tube must be composed of a material that shrinks to a predetermined diameter by heating and that does not melt in the polyamide elastomer. Instead of the heat-shrinkable tube, a silicone tube having an inner diameter of about 0.5 mm to 0.8 mm may be used for covering and then melted by heating. Upon completion of the heating process, the heat-shrunk tube or the silicone tube was removed, and the tapered mandrel inserted into the tip of the shaft 102 was pulled out to complete the preparation of the base shaft 104 for fixing the balloon 103. Next, the balloon tube prepared by dip forming was fixed onto the base shaft 104. In particular, the balloon tube for forming the balloon was cut to a length of 12 mm, and a two-part urethane adhesive was thinly applied to the portion of the base shaft 104 within 5 mm from the proximal end. Bonding was first conducted at the proximal side (the left side of the drawing). After the proximal-side bonding portion was hardened, a 5-mm portion at the distal end of the balloon, i.e., the distal-side bonding portion, was retained, and stretching was conducted so that the 2-mm center portion was stretched to 5 mm. The stretched part was held between a nonslipping member, such as a rubber sheet, and the 5-mm portion at the distal end was bonded onto the base shaft 104 using a two-part adhesive. In bonding the distal end of the balloon, the portion to be bonded is preferably covered with a shrink tube to decrease the lumen of the balloon 103. In this manner, the adhesive can spread evenly. Upon completion of the bonding of the balloon 103, the mandrel inserted into the circular lumen 202 of the base shaft 104 was removed to form a guidewire lumen 106. Note that the sliding resistance with the guidewire can be minimized by forming a thin tube composed of a high slippage material, such as a high-density polyethylene, onto the inner wall of the circular lumen 202 of the dual lumen tube 201 in the course of forming the guidewire lumen 106 in the base shaft 104. In particular, a thin tube composed of a high density polyethylene is placed over the mandrel for forming the guidewire lumen 106, and the mandrel with the thin tube is inserted to the base shaft 104, followed by the same heating process. The outer wall of the high-density polyethylene is treated with oxygen plasma, and a two-part adhesive is thinly applied onto the processed outer wall to achieve sufficiently tight bonding.

The guidewire lumen 106 was arranged so as to extend from the distal end of the catheter and penetrate the interior of the balloon. A guidewire lumen port 107 was located at the proximal-side of the balloon 103.

A detachable hub for pressurizing and depressurizing the balloon 103 was mounted at the proximal end of the shaft 102 to prepare the intravascular temporary occlusion catheter 101 of the present invention.

### (EXAMPLE 2)

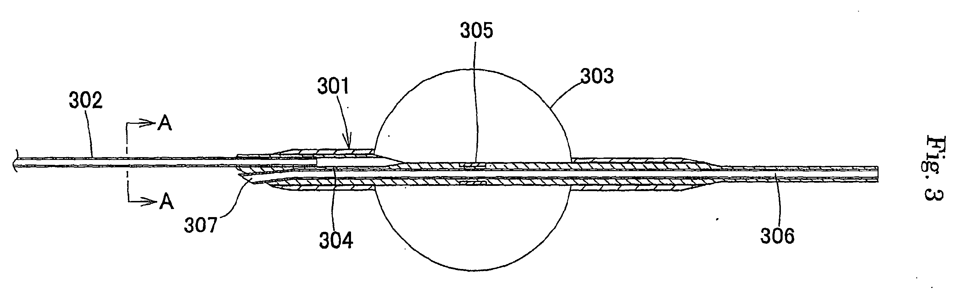

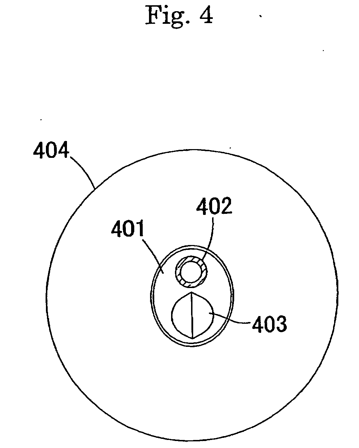

Fig. 3 shows another example of the present invention. The structure of a catheter 301 is basically the same as in EXAMPLE 1. In Fig. 3, reference numeral 302 denotes a shaft, 303 denotes a balloon, 304 denotes a base shaft, 305 denotes a radiopaque marker, 306 denotes a guidewire lumen, and 307 denotes a guidewire port. The difference from EXAMPLE 1 lies in that the balloon was composed of thermoplastic polyurethane Tecoflex EG85A manufactured by Thermedics, that methylene chloride was used as the solvent, and that a high-density polyethylene thin tube was used as the guidewire lumen tube for making the guidewire lumen. The guidewire lumen tube had a 1-mm projected portion at the guidewire port portion and this portion was crushed. Fig. 4 is a diagram viewed from the A-A cross-section of Fig. 3 in the direction toward the catheter distal end. In Fig. 4, reference numeral 401 denotes a base shaft, 402 denotes a shaft cross-section, 403 denotes a guidewire port, and 404 denotes a balloon.

### (EXAMPLE 3)

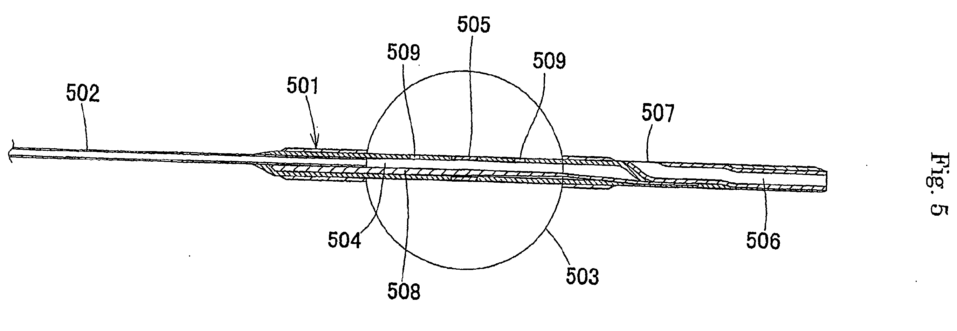

In EXAMPLE 3, the guidewire lumen for tracking the guidewire was formed at the distal-end side of the balloon. Fig. 5 shows the structure of this example. A shaft 502 of a catheter 501 was a metal tube composed of SUS 304 stainless steel having the same dimensions as in EXAMPLE 1. A SUS 304 stainless steel core wire 508 having a proximal-side outer diameter of 0.2 mm and a length of 18 mm was disposed at the distal-end portion of the shaft 502. The reason for providing this core wire 508 is to gradually change the stiffness from the shaft 502 to the catheter distal-end portion so that a guidewire lumen 506 is formed at the distal-end portion of the shaft 502. The core wire 508 may be tapered so that the wire becomes gradually thinner toward the distal end. In this manner, the stiffness of the catheter distal-end portion can gradually decrease. In this example, the core wire 508 was adjusted so that the outer diameter at the most distal end was 0.15 mm. Although YAG laser processing is the simplest and most effective way to bond the shaft 502 to the core wire 508, an adhesive may also be used instead. A YAG laser ML-2051A manufactured by Miyachi Technos Corporation was used as the YAG laser. The shaft 502 was arranged such that the periphery of a 3-mm distal-end portion of the shaft 502 overlapped the core wire 508. Pulse laser radiation was applied at three locations at an interval of 1 mm to bond the shaft 502 to the core wire 508. A balloon 503 was formed by a dip-forming process using a solution containing 5 percent by weight of a thermoplastic polyurethane Tecothane TT-1085A manufactured by Thermedics (U.S.) in a tetrahydrofuran (THF) solvent. The dip-forming process was conducted by dipping a tetrafluoroethylene-coated mandrel, i.e., a core, having an outer diameter of 1.0 mm in the prepared solution and withdrawing the mandrel so as to form a balloon tube on the mandrel. The dipping and withdrawing of the mandrel were repeated until the thickness of the tube became 80 µm, which was sufficient for use as the balloon 503. The tube was cut to a length of 8 mm to obtain a balloon tube. Next, a base shaft 504 for fixing the balloon 503 and forming the guidewire lumen 506 was processed. In particular, a tube having an outer diameter of 0.75 mm and an inner diameter of 0.60 mm was extrusion-molded using a polyamide elastomer Pebax 7033 and was cut to a length of 22 mm. A first end of the tube was attached to a distal end of the shaft 502, and a second end of the tube defined the guidewire lumen 506 and the distal-end tip of the catheter 501. A radiopaque marker 505 composed of a platinum-iridium alloy was fixed in advance at the center of the portion of the base shaft where the balloon 503 was to be fixed. The radiopaque marker 505 may have an outer diameter of 0.84 mm, an inner diameter of 0.76 mm and a length of 1 mm, and can be fixed onto the base shaft 504 by mechanically deforming the radiopaque marker 505. In this example, the radiopaque marker 505 was fixed at a position approximately 7.5 mm away from the proximal end (the end to which the metal shaft is fixed) of the base shaft. Next, the distal end of the base shaft 504 was processed to form the guidewire lumen 506. In particular, a side hole was first formed at a position 5 mm from the second end of the base shaft 504. A preliminarily prepared tube composed of a polyamide elastomer Pebax 7033 having an outer diameter of 0.50 mm and an inner diameter of 0.40 mm was cut to a length of 10 mm. Into this 10-mm guidewire lumen tube, a tetrafluoroethylene-coated mandrel having an outer diameter of 0.40 mm was inserted. The mandrel with the guidewire lumen was inserted into the base shaft 504 toward the distal end from the side opening formed in the base shaft 504. The distal end of the guidewire lumen tube was adjusted so as to protrude from the distal end of the base shaft 504 by 2 mm. The reason for such an adjustment is to easily process the most distal-end portion of the catheter 501 into a tapered shape by a thermal process, as is described below. While retaining the guidewire lumen tube in the base shaft 504, a tetrafluoroethylene-coated mandrel having an outer diameter of 0.15 mm was inserted from the proximal end of the base shaft 504 to a position 7 mm from the distal end of the base shaft 504. Another tetrafluoroethylene-coated mandrel having an outer diameter of 0.45 mm is inserted from the distal end of the base shaft 504 to a position 8 mm from the distal end. A heat-shrinkable tube was provided to wrap the region within 10 mm from the distal end of the base shaft 504 and heated to combine the base shaft 504 and the guidewire lumen tube by melting. After sufficient cooling, the heat-shrunk tube and the mandrels were removed. Next, two balloon expansion side holes 509 having an inner diameter of 0.2 mm were respectively formed by laser at the two sides of the radiopaque marker 505 at positions 1 mm from both ends of the radiopaque marker 505. A low energy carbon dioxide laser is sufficient for forming the holes. Next, a tetrafluoroethylene-coated mandrel having an outer diameter of 0.58 mm was inserted from the proximal side of the base shaft 504 up to the position where the guidewire lumen tube was melt-bonded. A two-part urethane adhesive was applied in a thin layer covering the region from 13 mm to 16 mm from the distal end of the base shaft 504. The two-part urethane adhesive used in this example was the same as that in EXAMPLE 1. The balloon tube prepared in advance was inserted from the proximal end of the base shaft 504, so that the distal end of the balloon tube overlapped the region of the base tube where the adhesive was applied, thereby bonding the distal end of the balloon 503. After the bonded portion was hardened, a proximal-side balloon-bonding portion having a length of 3 mm extending from the proximal end of the balloon was retained, and the balloon was stretched so that the center portion was stretched from 2 mm to 5 mm. The stretched portion was held with a nonslipping member, and the 3-mm proximal-end portion was bonded to the base shaft 504 with a two-part adhesive. In bonding the proximal-end portion of the balloon, the portion to be bonded is preferably wrapped with a shrinkable tube just like EXAMPLE 1, and heated to decrease the lumen size of the bonding portion of the balloon 503. In this manner, the adhesive can spread evenly. Upon completion of the bonding of the balloon 503, the mandrel placed in the base shaft 504 was removed, and the shaft 502 was bonded. In particular, a two-part urethane adhesive was applied on the distal end of the core wire 508 and the outer wall of a 5-mm portion of the shaft 502 extending from the end of the shaft 502 to which the core wire 508 was attached; subsequently, the base shaft 504 bonded with the balloon 503 was inserted into the distal end of the shaft 502. The shaft 502 was inserted such that the distal end of the core wire 508 was inserted in the hole formed during the melt bonding of the guidewire lumen tube of the base shaft 504 by the insertion of the mandrel having a diameter of 0.15 mm. After the adhesive was hardened, a detachable hub the same as that in EXAMPLE 1 was mounted to obtain the inventive intravascular temporary occlusion catheter 501.

### (EXAMPLE 4)

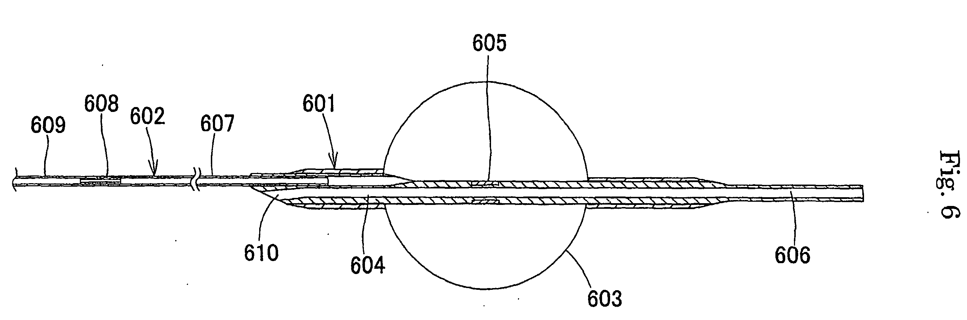

Fig. 6 shows another example of the present invention. The structure of a catheter 601 is basically the same as in EXAMPLE 1. The difference from EXAMPLE 1 lies in that a shaft 602 is constituted from a distal-end shaft 607 composed of a NiTi alloy, i.e., a superelastic metal, and a proximal-end shaft 609 composed of SUS 316 stainless steel. The superelastic metal tube (the distal-end shaft 607) had a length of 400 mm, an outer diameter of 0.35 mm, and an inner diameter of 0.28 mm. The distal-end shaft 607 was bonded to the proximal-end shaft 609 via an extension tube 608 using a two-part urethane adhesive, as shown in Fig. 6. The extension tube 608 had a length of 20 mm, an outer diameter of 0.25 mm, and an inner diameter of 0.18 mm and was composed of SUS 316 stainless steel. In bonding the extension tube 608 to these shafts, appropriate care must be taken to prevent the adhesive from flowing into the lumens of the shafts. In order to avoid the formation of a step difference in the bonded portion, a silicon tube having an inner diameter of 0.25 mm and an outer diameter of 5.0 mm was disposed near the bonding portion in advance, moved to the bonded portion at the same time as the bonding, and left there until the adhesive had hardened. The silicon tube was then removed after the adhesive had hardened. The processing of a base shaft 604 and the bonding of a balloon 603 were conducted as in EXAMPLE 1. In Fig. 6, reference numeral 605 denotes a radiopaque marker, 606 denotes a guidewire lumen, and 610 denotes a guidewire port.

### (EXAMPLE 5)

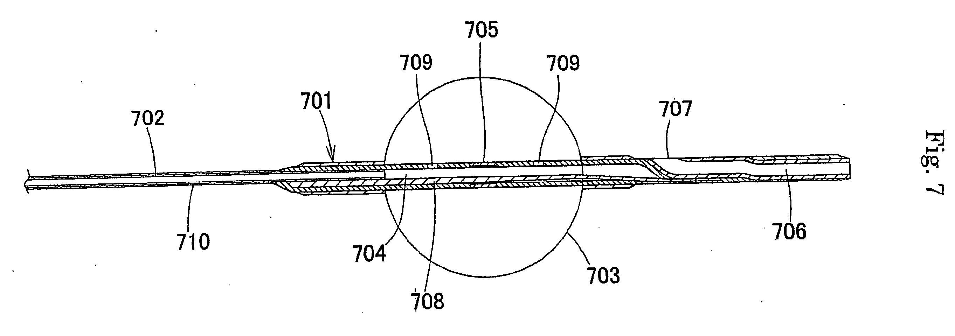

Fig. 7 shows another example of the present invention. The structure of a catheter 701 is basically the same as that in EXAMPLE 3. The difference from EXAMPLE 3 lies in that the outer surface of a metal shaft 702 is covered with a high-density polyethylene coating layer 710 and that the outer diameter of the coated shaft was 0.018 in. (0.4572 mm). The process of forming the high-density polyethylene coating layer 710 on the metal shaft 102 was as follows. A tube having an outer diameter of 0.60 mm and an inner diameter of 0.47 mm was prepared in advance by extrusion molding. The tube was cut to a length of 600 mm, placed around a metal tube, and passed through a die having an inner diameter of 0.45 mm heated to 130°C at a rate of 1 mm/sec so that the high-density polyethylene coating layer 710 was formed on the metal tube while limiting the outer diameter to 0.45 mm. The high-density polyethylene coating layer 710 was cut to an appropriate length with a razor blade or the like. In Fig. 7, reference numeral 703 denotes a balloon, 704 denotes a base shaft, a 705 denotes a radiopaque marker, 706 denotes a guidewire lumen, 707 denotes a guidewire port, 708 denotes a core wire and 709 denotes balloon expansion side holes.

### (EXAMPLE 6)

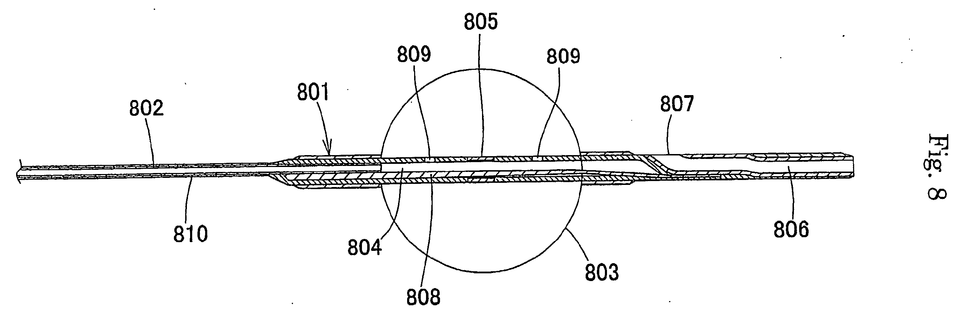

Fig. 8 shows another example of the present invention. The structure of a catheter 801 is basically the same as that in EXAMPLE 3. The difference from EXAMPLE 3 lies in that a metal shaft 802 had an outer diameter of 0.33 mm and an inner diameter of 0.26 mm and that the outer surface of the metal shaft 802 was covered with a polytetrafluoroethylene (PTFE) thin layer 810. The PTFE thin layer 810 was formed by a typical coating process including spraying and heating steps. In Fig. 8, reference numeral 803 denotes a balloon, 804 denotes a base shaft, 805 denotes a radiopaque marker, 806 denotes a guidewire lumen, 807 denotes a guidewire port, 808 denotes a core wire, and 809 denotes balloon expansion side holes.

### Industrial Applicability

As is described above, the intravascular temporary occlusion balloon catheter according to the present invention can itself function as a guidewire and has superior maneuverability compatible with highly tortuous and branched blood vessels such as coronary or cerebral arteries. Moreover, it can be inserted into a blood vessel over a guidewire and can adequately reach the peripheral region of the blood vessel.

## Claims

1. An intravascular temporary occlusion balloon catheter comprising a balloon comprising a highly tensile material having an elongation at break of 300% to 1,100% and a shaft composed of a highly elastic material and having an outer diameter in the range of 0.014 in. (0.3556 mm) to 0.018 in. (0.4572 mm) and a bending modulus of at least 1 GPa, wherein a lumen for tracking the guidewire is provided at a catheter distal-end portion only.

2. The intravascular temporary occlusion balloon catheter according to claim 1, wherein the lumen for tracking the guidewire crosses the interior of the balloon.

3. The intravascular temporary occlusion balloon catheter according to claim 2, wherein the lumen for tracking the guidewire has a proximal-side guidewire port located at a position within 10 mm from the proximal end of the inflated balloon.

4. The intravascular temporary occlusion balloon catheter according to claim 2 or 3, wherein the guidewire port is closed when no guidewire is present in the guidewire port.

5. The intravascular temporary occlusion balloon catheter according to claim 1, wherein the lumen for tracking the guidewire is located at the distal side of the balloon.

6. The intravascular temporary occlusion balloon catheter according to any one of claims 1 to 5, wherein the shaft comprises a material selected from the group consisting of SUS 304, SUS 316, and SUS 316L stainless steel.

7. The intravascular temporary occlusion balloon catheter according to any one of claims 1 to 6, wherein the shaft comprises a superelastic metal at least in the distal side.

8. The intravascular temporary occlusion balloon catheter according to any one of claims 1 to 7, wherein the outer surface of the shaft is covered with a thin resin layer comprising tetrafluoroethylene or polyethylene or a hydrophilic coating layer.

9. The intravascular temporary occlusion balloon catheter according to any one of claims 1 to 8, further comprising a radiopaque marker for identifying the position of the catheter by radioscopy, the radiopaque marker being disposed at least in the interior of the balloon.

10. The intravascular temporary occlusion balloon catheter according to any one of claims 1 to 9, wherein the balloon catheter comprises thermoplastic polyurethane, silicone, or natural rubber.
